# EUROPEAN PATENT SPECIFICATION

(11) **EP 2 005 990 B1**
(45) Date of publication and mention of the grant of the patent: **28.08.2013**
(21) Application number: 07741173.4
(22) Date of filing: 06.04.2007
(51) Int. Cl.: A61M 37/00, A61B 17/20, A61K 9/00, A61K 47/34

(54) **MICRONEEDLE DEVICE AND TRANSDERMAL ADMINISTRATION DEVICE PROVIDED WITH MICRONEEDLES**
MIKRONADELVORRICHTUNG UND TRANSDERMALE VERABREICHUNGSVORRICHTUNG MIT MIKRONADELN
APPAREIL À MICRO-AIGUILLES ET APPAREIL D'ADMINISTRATION TRANSDERMIQUE À MICRO-AIGUILLES

(30) Priority: 07.04.2006 JP 2006106995
(43) Date of publication of application: 24.12.2008
(73) Proprietor: Hisamitsu Pharmaceutical Co., Inc., Tosu-shi, Saga 841-0017 (JP)
(72) Inventor: TOKUMOTO, Seiji, Tsukuba-shi Ibaraki 305-0856 (JP); MATSUDO, Toshiyuki, Tsukuba-shi Ibaraki 305-0856 (JP); KUWAHARA, Tetsuji, Tsukuba-shi Ibaraki 305-0856 (JP)
(74) Representative: Westendorp, Michael Oliver
(86) International application number: PCT/JP2007/057737
(87) International publication number: WO 2007/116959

(56) References cited:
- EP-A1- 1 790 375
- WO-A1-2006/016647
- WO-A1-2006/055799
- WO-A2-2005/044366
- JP-A- 2001 316 215
- US-A1- 2005 031 676
- US-A1- 2005 031 676
- PÉRENN GBP ES F ET AL: "Sharp beveled tip hollow microneedle arrays fabricated by LIGA and 3D soft lithography with polyvinyl alcohol; Sharp beveled tip hollow microneedle arrays", JOURNAL OF MICROMECHANICS & MICROENGINEERING, INSTITUTE OF PHYSICS PUBLISHING, BRISTOL, GB, vol. 16, no. 3, 1 March 2006 (2006-03-01), pages 473-479, XP020104929, ISSN: 0960-1317, DOI: DOI:10.1088/0960-1317/16/3/001
- CHAN LAI WAH ET AL: "Evaluation of permeability and mechanical properties of composite polyvinyl alcohol films", CHEMICAL AND PHARMACEUTICAL BULLETIN (TOKYO), vol. 47, no. 10, October 1999 (1999-10), pages 1412-1416, XP002626065, ISSN: 0009-2363

## Description

### Technical Field

The present invention relates to a microneedle device having a plurality of microneedles on a substrate, which are capable of piercing a skin for administering a drug through a skin, and a transdermal drug administration apparatus with microneedles.

### Background Art

The method of administering a drug by applying a drug containing patch on the skin, and allowing the drug to penetrate into the skin from the patch, has been conventionally used in general. On the other hand, the method of administering drugs with help of electrical energy, such as iontophoresis (Journal of Pharmaceutical Sciences, Vol. 76, p. 341, 1987) and electroporation (National Publication of International Patent Application No. 03-502416; Proc. Natl. Acad. Sci. USA, Vol. 90, p. 10504-10508, 1993), have been developed as methods of promoting drug uptake through the skin or mucosa. The applications of iontophoresis and electroporation are looked forward to with high expectations, as methods of promoting transdermal or transmucosal drug absorption.

Apart from this, microneedle-equipped devices are known, for instance, from National Publication of International Patent Application No. 2000-512529 (Patent document 1) as devices that increase transdermal flux by mechanically piercing the skin before releasing the transdermal drug. This kind of technology has become of particular interest because in recent years there have been many advances in pain reduction and improvement of transdermal permeability. The device has a sheet with a plurality of openings, a plurality of microblades that are integrated with the sheet and extend downwards from the sheet, and means of holding the device in position on the body surface. In this case, the drug product placed in the drug reservoir is in the form of a viscous gel. Also, the National Publication of International Patent Application No. 2004-501724 (Patent document 2) discloses transdermal delivery means of hormonal substances in which pain reduction and assured delivery of a hormonal substance are achieved by specifying the length of a number of small gauge needles at about 300 µm to 2 mm, and the needle insertion depth as about 250 µm to 2 mm.

There have been further advances in recent years. Japanese Patent Laid-Open No. 2003-238347 (Patent document 3) proposes the installation, on a substrate, of a columnar pile mainly made of saccharides that dissolve and get cleared in the living body. The functional micropile creates passages that reach the horny layer of the skin and enables delivery of the functional substance specifically to the horny layer, through a simple painless procedure, safely, and effectively. Japanese Patent Laid-Open No. 2004-65775 (Patent document 4) discloses a device having needle-like structure elements having a thin film, through which the needle part of the needle-like structure element can penetrate, present on the needle tip part of the needle-like structure element, and an adhesive is applied to the surface of this thin film.

Furthermore, in recent years, various advances have been made in the techniques of coating microneedles. National Publication of International Patent Application No. 2004-504120 (Patent document 5) discloses an interface having microneedles, wherein the skin-piercing member is coated with a reservoir medium, or is itself made of the reservoir medium, as a device for inoculating a vaccine through the skin. It is reported that biodegradable sugars (lactose, raffinose, trehalose, and sucrose), which can easily release the drug contained in them by getting dissolved, are preferable as the reservoir medium. National Publication of International Patent Application No. 2004-528900 (Patent document 6) describes the selection of the coating carrier, for the microprojection array used for transdermal administration of vaccines, etc, from among human albumin, polyglutamic acid, polyasparaginic acid, polyhistidine, pentosan polysulfuric acid, and polyamino acids. This coating carrier also rapidly dissolves when it passes through the skin and thereby releases the useful active substance. WO2005/016440 (Patent document 7) discloses coating carriers containing a polymer such as hydroxymethyl cellulose (HPMC), hydroxypropyl cellulose, dextran, polyvinyl alcohol, and polyethylene oxide. Here, because the coating carrier has fluidity, with a viscosity of 3 to 500 cps, by making some arrangements on the surface of the needles, the needle tips are automatically coated with the coating carrier. It is mentioned that because of this there is no need for a coating operation and a long period of effectiveness can be achieved. However, in this case, as the coating carrier is forced through the skin, it is difficult to control it, and there is some doubt about its practical utility.

The method of coating the microneedles of the needle structures with the drug or coating agent as described above has been mostly used for administering only small quantities of substances like vaccines because the quantity of drug that can be administered is limited to very small amounts. Particularly in the case of low molecular weight active compounds that generally do not show their action unless a significant amount is administered into the living body, the conventional type of coating carrier assumes a dissolved state after passing through the skin. So, the useful drug is released in one go and an effective level of the drug's effect cannot be sustained for a long time. For this reason, the coating technique was considered unsuitable for use with low molecular weight compounds.
Patent document 1: National Publication of International Patent Application No. 2000-512529
Patent document 2: National Publication of International Patent Application No. 2004-501724
Patent document 3: Japanese Patent Laid-Open No. 2003-238347
Patent document 4: Japanese Patent Laid-Open No. 2004-65775
Patent document 5: National Publication of International Patent Application No. 2004-504120
Patent document 6: National Publication of International Patent Application No. 2004-528900
Patent document 7: WO2005/016440

WO 2005/044366 discloses microneedle systems for vaccine delivery.

### Disclosure of the Invention

The purpose of the present invention is, therefore, to provide a microneedle device having a coating, which is effective even with a low molecular weight active compound and can sustain the effect of the drug for a long period of time, and a transdermal drug administration apparatus with microneedles.

To achieve the aforesaid purpose, various water-soluble polymers were examined for use as coating carrier for microneedles. As a result, it was found that polyvinyl alcohols, among them, particularly those with saponification degree 94.5 mol% or more, had superior coating property, and better skin permeability of the drug, compared to other water-soluble polymers, which led to the completion of the present invention according to claim 1.

Besides this, the coating carrier with polyvinyl alcohol with saponification degree 94.5 mol% or more, once fixed to the target material, does not dissolve even in an aqueous solvent, and retains its film shape. Therefore, it became clear that clearly unlike hitherto known soluble drug-releasing coating carriers, the new coating carrier functions not only as the drug carrier but also acts as the drug permeation route through a microneedle interface (microneedle device).

In short, the microneedle device of the present invention comprises a plurality of microneedles on a substrate, which are capable of piercing a skin, and the surface of the microneedles and/or the substrate is partly or entirely coated in fixed state with a coating carrier containing polyvinyl alcohol. The coating carrier preferably maintains fixed state, without completely dissolving even after the transdermal application, and the polyvinyl alcohol has a saponification degree of 94.5 mol% or more. The coating carrier can contain a drug.

The transdermal drug administration apparatus with microneedles of the present invention has a microneedle device comprising a plurality of microneedles on a substrate, which are capable of piercing a skin, and the surface of the microneedles and/or the substrate is partly or entirely coated in fixed state with a coating carrier containing a polyvinyl alcohol and a drug. The apparatus can further comprise a dissolving solution reservoir containing a drug solution or a dissolving solution for drug dissolution above the microneedle device.

Further, the transdermal drug administration apparatus with microneedles of the present invention has a microneedle device comprising a plurality of microneedles on a substrate, which are capable of piercing a skin, and a drug retainer retaining a drug and arranged above the microneedle device, and the surface of the microneedles and/or the substrate is partly or entirely coated in fixed state with a coating carrier containing polyvinyl alcohol. The apparatus can further comprise a dissolving solution reservoir containing a drug solution or a dissolving solution for drug dissolution above the drug retainer. The apparatus can further comprise an electrode for supplying electrical energy from the outside, or a sonic transducer for supplying sonic vibration energy from the outside. The polyvinyl alcohol has a saponification degree of 94.5 mol% or more.

A method of coating a microneedle device of the present invention comprising a plurality of microneedles on a substrate, which are capable of piercing a skin, comprises the steps of coating the surface of the microneedles and/or the substrate partly or entirely with a coating carrier containing polyvinyl alcohol, and drying and fixing the coating carrier thereto. The coating carrier can contain a drug. Also, it is preferable that, before fixing coating carrier, the polyvinyl alcohol has a viscosity of 1 to 60,000 cps, and a mean degree of polymerization of 200 to 3500.

According to the present invention, by coating microneedles with a coating carrier containing polyvinyl alcohol, in transdermal administration of the physiologically active substance (drug) using the microneedle device, we can obtain a microneedle device, which shows good skin permeability and sustainability of the drug effect of low molecular weight physiologically active substances (drugs), achievements hitherto considered difficult, and a transdermal drug administration apparatus with microneedles.

### Brief Description of the Drawings

Figure 1 is a diagram of an example of microneedle devices of the present invention; (a) is a diagonal view; (b) is a cross-sectional diagram at A-B of (a); and (c) and (d) are cross-sectional diagrams at A-B of other examples;
Figure 2 is a diagram of an example of a transdermal drug administration apparatus with microneedles of the present invention;
Figure 3 is a diagram of another example of a transdermal drug administration apparatus with microneedles of the present invention;
Figure 4 is a diagram of another example of a transdermal drug administration apparatus with microneedles of the present invention;
Figure 5 is a graph showing the results of measurement in Example 1;
Figure 6 is a graph showing the results of measurement in Example 2;
Figure 7 is a graph showing the results of measurement in Example 3;
Figure 8 is a graph showing the results of measurement in Example 4; and
Figure 9 is a graph showing the results of measurement in Example 5.

### Description of Symbols

- 1: Coating
- 5, 50: Microneedle device
- 6, 51: Microneedle
- 7, 52: Opening (solution passage)
- 8, 53: Microneedle substrate
- 10: Drug
- 11: Absorbent
- 12: Adhesive layer
- 13: Wall member
- 14: Opening
- 15: Support
- 16: Dissolving solution
- 17: Protruding portion
- 18: Dissolving solution reservoir
- 20: Diaphragm
- 31: Absorbent
- 32: Drug retainer
- 41: Pad portion

### Best Mode for Carrying Out the Invention

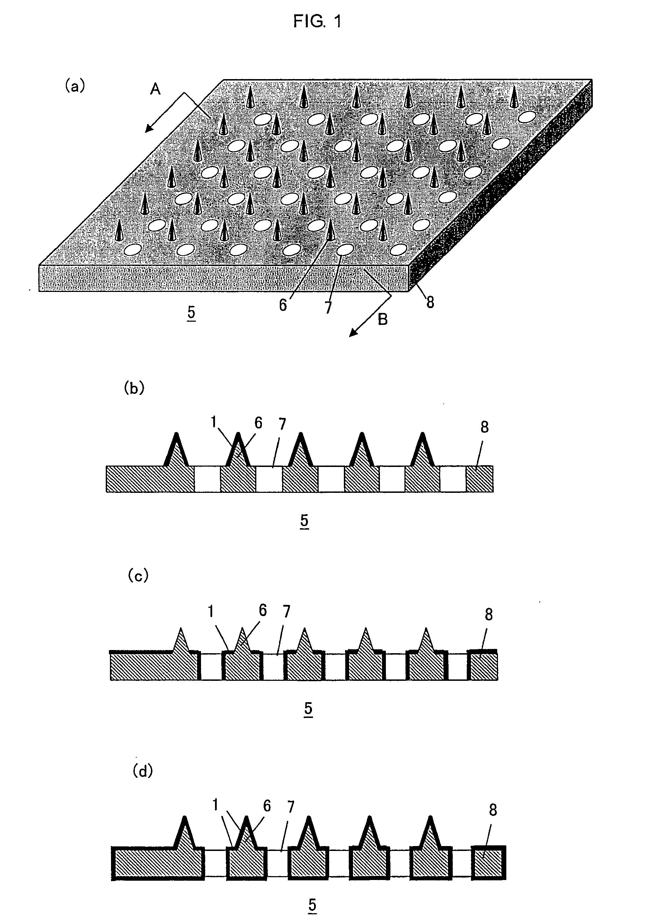

Figure 1 shows an example of microneedle devices of the present invention, where (a) is a diagonal view, (b) is a cross-sectional diagram at A-B of (a), and (c) and (d) are cross-sectional diagrams at A-B of other examples. As shown in Figure 1 (a), the microneedle device (interface) 5 of the present invention has a microneedle substrate 8, and a plurality of microneedles 6 that can pierce the skin or mucosa and are arranged in a 2-dimensional array. The microneedle substrate 8 has a plurality of openings 7, arranged corresponding to the microneedles 6. In this example, the microneedles 6 have a conical shape, but the invention is not limited to this shape. The microneedles can be polygonal pyramids such as square pyramids, or any other shape. Although a plurality of microneedles 6 and a plurality of openings 7 are arranged alternately in a square lattice pattern in this example, the present invention is not limited to this arrangement. Further, although the number of microneedles 6 and openings 7 shown in the Figure are in the ratio of 1:1, the present invention is not limited to this, and covers devices without the openings 7 also.

In the present invention, the surface of the microneedles 6 and/or the substrate 8 is partly or entirely (including the inner surfaces of the openings 7) coated in fixed state with a coating carrier containing polyvinyl alcohol. Here, the microneedle device of the present invention is not limited to those used for drug administration. However, in this example, the drug can be contained in the coating carrier. Also, the drug can be supplied to the microneedle device by some other means than including the drug in the coating carrier. The coating 1 is positioned, for instance, on the surface of each microneedle 6 as shown in Figure 1 (b). The coating 1 can be positioned only partial rather than on the entire surface of the microneedle 6. Also, as shown in Figure 1 (c), the coating 1 can be positioned on a part (including the inner surfaces of the openings 7) of the substrate 8. Furthermore, the coating 1 can be positioned on the entire surface (including the inner surfaces of the openings 7) of the substrate 8, as shown in Figure 1(d). Although not shown in the Figure, the coating 1 need not be positioned on the inner surfaces of the openings 7 also. When the microneedle substrate surface on which the microneedles 6 are positioned as shown in Figure 1(a) is pressed over the skin, and the liquid for dissolving the drug, or the drug-containing solution, is fed from the other side of the substrate at the time of use, the liquid flows out through each of the openings 7 and gets transferred to each microneedle 6, and the drug gets transdermally absorbed. Here, it is not essential to have the openings 7. The fluid may be supplied to the microneedle 6 by some other means that does not involve the use of the openings 7.

A microneedle (the needle part) has a microstructure, and its size (height) is preferably 50 µm to 1000 µm, more preferably 50 µm to 500 µm. Here, "microneedle" means a pointed structure, and in a broad sense, it means a needle-shaped structure or a structure including a needle-shaped structure, but it is not limited to a simple needle shape. Also, in some structures, the tip may not be pointed. So, microneedles are not restricted to those with sharp tips only. The substrate is a platform for supporting the microneedles (needle parts), and there are no particular limitations on its shape. The material of the microneedles can be silicon, silicon dioxide, ceramics, metals (stainless steel, titanium, nickel, molybdenum, chromium, cobalt, etc), and plastics, polylactic acid, polyglycolic acid, and their copolymers, etc. Examples of methods of producing microneedles include wet etching process or dry etching process of a silicon substrate, precision machining (electrical discharge machining, laser machining, dicing, etc) of metals and plastics, machine cutting, extrusion molding, emboss processing, etc. The microneedles and substrates can be shaped in an integrated manner using these methods of processing. The microneedles can be hollow. The microneedles may be made hollow by secondary processing, such as laser machining, after they are prepared.

The coating carrier used on the microneedles in the present invention contains polyvinyl alcohol of saponification degree of 78 to 100 mol%. In particular, those with a saponification degree of 94.5 mol% or more, especially those that are fully saponified grades, i.e., with a high saponification degree are more preferable. For instance, in the case of PVA117 (KURARAY CO., LTD.), fully saponified grades have a saponification degree 97 mol% or more. Preferably, the polyvinyl alcohol has a mean degree of polymerization of 200 to 3500, more preferably 1000 to 2000. When the mean degree of polymerization is less than 500, the amount of permeation tends to decrease.

The content of polyvinyl alcohol in the coating carrier is 1 to 20 wt.%, 3 to 8 wt.% being particularly preferable. To prevent dripping, the coating carrier is required to have a viscosity of about 1 to 60,000 cps, more preferably 30 to 30,000 cps, most preferably 100 to 20,000 cps.

The mean thickness of the coating is less than 50 µm, most preferably less than 25 µm, 0.1 to 10 µm for example. The thickness of the coating is generally the mean thickness of the coating measured on the surface of the microneedles after drying. In general, the thickness of the coating can be increased by applying more than one coat of the coating carrier, and drying between successive coats. The coating is made by applying the coating carrier on the surface of the microneedles by a known method, and drying. Also, the coating can be applied on the inner surfaces of hollow needle structures of the microneedles, and the lower surface, side surfaces, and upper surface of the microneedle substrate, and the inner surfaces of the openings made on the substrate.

The physiologically active substance (drug) used in the present invention is a low molecular weight compound, with no particular limitation. Low molecular weight means of molecular weight 1000 or less. Compounds with molecular weight 100 to 800 are particularly suitable. There is no particular limitation on the type of drug, other than the low molecular weight. Examples include hypnotics and sedatives (flurazepam hydrochloride, rilmazafon hydrochloride, phenobarbital, amobarbital, etc), antipyretic, anti-inflammatory and analgesic agents (butorphanol tartrate, perisoxal citrate, acetaminophen, mefenamic acid, diclofenac sodium, aspirin, alclofenac, ketorpofen, flurbiprofen, naproxen, piroxicam, pentazosin, indomethacin, glycol salicylate, aminopirin, loxoprofen, etc), steroidal antiinflammatory agents (hydrocortisone, prednisolone, dexamethasone, betamethasone, etc), analeptic stimulants (methamphetamine hydrochloride, methylphenidate hydrochloride, etc), psychotropic drugs (imipramine hydrochloride, diazepam, sertraline hydrochloride, fulvoxamine maleate, paroxetine hydrochloride, citalopram hydrobromide, fuloxetine hydrochloride, alprazolam, haloperidol, clomipramine, amitriptilin, decipramine, amoxapine, maprotylin, mianserin, setiptilin, trazadone, lofepramine, milnaciplan, duroxetine, venlafaxine, chlorpromazine hydrochloride, thioridazine, diazepam, meprobamate, etizolam, etc), hormone formulations (estradiol, estriol, progesterone, norethisterone acetate, metelonon acetate, testosterone, etc), local anesthetics (lidocaine hydrochloride, procaine hydrochloride, tetracaine hydrochloride, dibucaine hydrochloride, propitocaine hydrochloride, etc), urological drugs (oxybutynine hydrochloride, tamsulosin hydrochloride, propiverin hydrochloride, etc), skeletal muscle relaxants (tizanidine hydrochloride, eperisone hydrochloride, pridinol mesylate, suxamethonium hydrochloride, etc), reproductive system drugs (ritodrine hydrochloride, meluadrine tartrate), antiepileptic drugs (sodium valproate, clonazepam, carbamazepine, etc), autonomous nervous system drugs (carpronium chloride, neostigmine bromide, bethanechol chloride, etc), anti-Parkinson drugs (pergolide mesylate, bromocriptine mesylate, trihexiphenidyl hydrochloride, amantazine hydrochloride, ropinirole hydrochloride, talipexol hydrochloride, cabergoline, droxidopa, piperiden, selegiline hydrochloride, etc), diuretics (hydroflumethiazide, furosemide, etc), respiration promoters (lobeline hydrochloride, dimorpholamine, naloxone hydrochloride, etc), antimigraine drugs (dihydroergotamine mesylate, sumatriptan, ergotamine tartrate, flunaridine hydrochloride, cyproheptadine hydrochloride, etc), antihistamines (clemastine fumarate, diphenhydramine tannate, chlorphenylamine maleate, diphenylpyraline hydrochloride, promethazine, etc), bronchodilators (tolubuterol hydrochloride, procaterol hydrochloride, salbutamol sulfate, clenbuterol hydrochloride, fenoterol hydrobromide, terbutaline sulfate, isoprenaline sulfate, formoterol fumarate, etc), cardiac stimulants (isoprenaline hydrochloride, dopamine hydrochloride, etc), coronary vasodilators (diltiazem hydrochloride, verapamyl hydrochloride, isosorbide nitrate, nitroglycerin, nicorandil, etc), peripheral vasodilators (nicametate citrate, trazoline hydrochloride, etc), antismoking drugs (nicotine, etc), circulatory organ agents (flunarizine hydrochloride, nicardipine hydrochloride, nitrendipine, nisoldipine, felodipine, amlodipine besylate, nifedipine, nilvadipine, manidipine hydrochloride, benedipine hydrochloride, enalapril maleate, temocapril hydrochloride, alacepril, imidapril hydrochloride, cilazapril, lisinopril, captopril, trandolapril, perindopril erbumine, atenolol, bisoprolol fumarate, metoprolol tartrate, betaxolol hydrochloride, arotinolol hydrochloride, celiprolol hydrochloride, carvedilol, carteolol hydrochloride, bevantolol hydrochloride, valsartan, candesartan, cilexetil, losartan potassium, clonidine hydrochloride, etc), antiarrhythmic drugs (propranolol hydrochloride, alprenolol hydrochloride, procainamide hydrochloride, mexiletine hydrochloride, nadolol, disopyramid, etc), antineoplastic agents (cyclophosphamide, fluorouracil, tegafur, procarbazine hydrochloride, ranimustine, irinothecan hydrochloride, fluridine, etc), antilipidemia drugs (pravastatin, simvastatin, bezafibrate, probucol, etc), hypoglycemic agents (glibenclamide, chlorpropamide, tolubutamide, glymidine sodium, glybzole, buformin hydrochloride, etc), peptic ulcer drugs (proglumide, cetraxate hydrochloride, spizofurone, cimetidine, glycopyrronium bromide), choleretic drugs (ursodesoxycholic acid, osalmid, etc), eneterokinetic agents (domperidone, cisapride, etc), drugs for hepatic diseases (thiopronin, etc), antiallergy drugs (ketotifen fumarate, azelastine hydrochloride, etc), antiviral drugs (acyclovir, etc), antivertigo agents (betahistine mesylate, difenidol hydrochloride, etc), antibiotics (cephaloridin, cephdinyl, cephpodoxime proxetil, cefachlor, clarithromycin, erythromycin, methyl erythromycin, kanamycin sulfate, cycloserine, tetracycline, benzylpenicillin potassium, propicillin potassium, cloxacillin sodium, ampicillin sodium, bacampicillin hydrochloride, carbenicillin sodium, chloramphenicol, etc), anti-addiction drugs (cyanamide, etc), appetite suppressants (mazindol, etc), chemotherapy drugs (isoniazid, ethionamide, pyrazinamide, etc), blood coagulation accelerators (ticlopidine hydrochloride, warfarin potassium), anti-Alzheimer drugs (physostigmine, donepezyl hydrochloride, tacrin, arecoline, xanomelin, etc), serotonin receptor antagonist antinausea drugs (ondansetron hydrochloride, granisetron hydrochloride, ramosetron hydrochloride, azasetron hydrochloride, etc), gout drugs (colchicine, probenecid, sulfinpyrazone, etc),and narcotic analgesics (fentanyl citrate, morphine sulfate, morphine hydrochloride, codeine phosphate, cocaine hydrochloride, pethidine hydrochloride, etc). As long as the molecular weight is 1000 or less, physiologically active substances like vaccines, low molecular weight peptides, sugars, nucleic acids, etc also can be used.

These drugs can be used singly or in combinations of two or more, and drugs in the form of inorganic and organic salts are both naturally included, as long as they are pharmaceutically permissible. The drug is included in the coating carrier. It can also be supplied via the through-holes (openings) made on the microneedle substrate.

The liquid composition used for coating the microneedles is prepared by mixing the biocompatible carrier, the useful active substance to be delivered, and any coating adjuvant in some cases, with a volatile fluid. There is no particular limitation on the volatile fluid, but water, dimethylsulfoxide, dimethylformamide, ethanol, isopropyl alcohol and their mixtures can be used. Water is most preferable among these. The liquid coating solution or suspension can typically have 0.1 to 60 wt.% of the beneficial, low molecular weight, physiologically active substance concentration, the preferable concentration being 1 to 30 wt.%, more preferably 3 to 20 wt.%. "Fixed" here means that the coating carrier is almost uniformly attached to the object to be coated. Immediately after the coating, coating carrier is fixed under the dry state by a known method like air drying, vacuum drying, freeze-drying, or their combinations. But it need not remain to be fixed under the dry state after the transdermal administration because it might have a water content that is at equilibrium with the surroundings, or it may retain an organic solvent, etc.

Other adjuvants known to be used in drug formulations may be added, depending on the solubility and viscosity required in the coating, to the extent that has no harmful effect on the physical integrity of the dried coating.

The microneedle device of the present invention transdermally delivers a physiologically active substance (drug) via the plurality of microneedles coated with a fixed solid or gel-form coating containing a useful physiologically active substance (drug). Various forms can be imagined for the apparatus. For instance, the microneedle substrate can have more than one solution passage (opening). Moreover, it can also have a sheet-shaped reinforcing member having one or more solution passage (openings). Further, a pad portion placed above the microneedle substrate, and a dissolving solution reservoir that contains a dissolving solution for dissolution drug, and is placed above the pad portion, can also be provided. The microneedle interface provided with such a dissolving solution reservoir is disclosed, for instance, in WO03/084595A1. It is also possible for the transdermal drug administration apparatus to be a blister type transdermal drug administration apparatus with microneedles in which the seal of the aforementioned dissolving solution reservoir breaks when the dissolving solution reservoir is pressed, and the dissolving solution is supplied to the pad portion, while at the same time, the microneedles pierce the horny layer of the skin, and thereby the drug dissolved in the dissolving solution is absorbed transdermally. An example of a blister type apparatus will be described hereinafter.

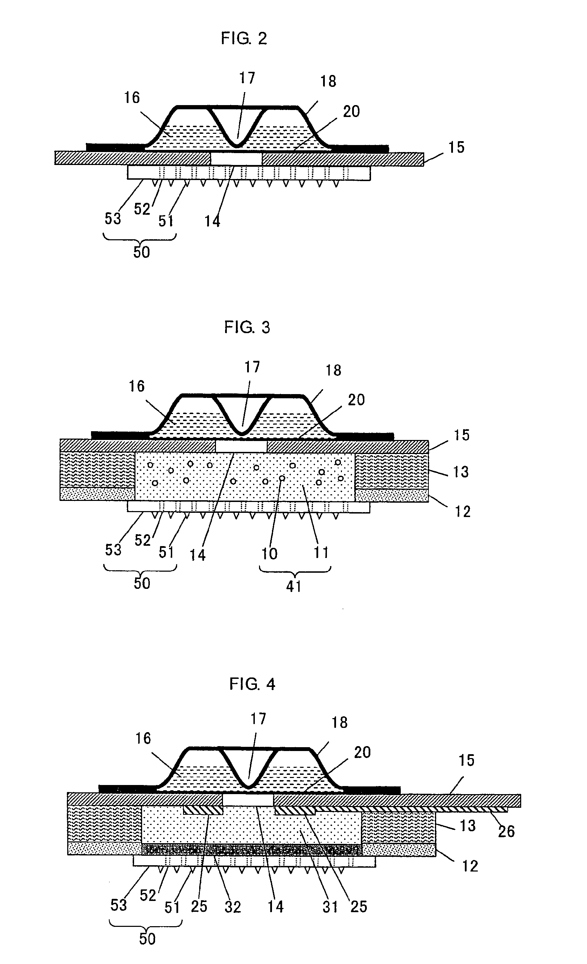

Figure 2 is a diagram showing an example of a transdermal drug administration apparatus with microneedles of the present invention. This apparatus has a microneedle device 50, having a microneedle substrate 53 with a plurality of microneedles 51 that can pierce the skin, and a dissolving solution reservoir 18 that is positioned above the microneedle device 50 and contains the dissolving solution 16 for dissolving the drug. In this example, at least one solution passage (opening) 52 is formed on the microneedle substrate 53. In this example, the microneedle device 50 is coated in fixed state with a coating carrier, containing polyvinyl alcohol and/or a drug. The coating is, for instance, placed on any site of the outer surface, or inner surface of the hollow passage, of the microneedle 51; or the upper surface, lower surface, side surfaces, or the inner surfaces of the solution passage(s) 52, of the microneedle substrate 53; or more than one of these sites. At the time of its use, the apparatus is placed on the skin and the protruding portion 17 of the dissolving solution reservoir 18 is pressed down to break the diaphragm 20, which opens the seal of the dissolving solution reservoir 18. The dissolving solution 16 is thus supplied to the microneedle device 50 through the opening 14 formed on the support 15. As a result, the dissolving solution 16 is supplied to the microneedles 51 through the solution passage 52 formed on the microneedle substrate 53. At the same time, the microneedles 51 pierce the horny layer of the skin, and the drug in the coating, which is now dissolved by the dissolving solution, is absorbed transdermally.

Figure 3 is a diagram showing another example of a transdermal drug administration apparatus with microneedles of the present invention. This apparatus has, as shown in the Figure, a microneedle device 50 with a microneedle substrate 53 having a plurality of microneedles 51 that can pierce the skin, and at least one solution passage 52; a pad portion 41 positioned above the microneedle device 50; and a dissolving solution reservoir 18 positioned above the pad portion 41, which contains the dissolving solution 16 for dissolving the drug, and the seal of which can be broken by applying pressure. In this example, the microneedle device 50 is coated with a coating carrier containing polyvinyl alcohol which is firmly fixed thereto. The coating is, for instance, placed on any site of the outer surface, or inner surface of the hollow passage, of the microneedle 51; or the upper surface, lower surface, side surfaces, or the inner surfaces of the solution passages 52 of the microneedle substrate 53; or more than one of these sites. The pad portion 41 in this example is a drug retainer, which has an absorbent 11 that consists of a material that can absorb fluids, and the drug 10. Around the absorbent 11 is placed a wall member 13 having an adhesive layer 12 on its lower surface. A support 15 having opening 14 is placed on the absorbent 11 and wall member 13, and a diaphragm 20 is placed on this support 15. The diaphragm 20 can be formed separately from the dissolving solution reservoir 18 or be integrated with it. The dissolving solution reservoir 18 has a protruding portion 17 to make it easy to break the diaphragm 20. At the time of its use, the apparatus is fitted on the skin; the microneedles 51 face the surface of the horny layer of the skin, and the dissolving solution reservoir 18 is pressed down to break the diaphragm 20 with the protruding portion 17. This breaks the seal of the dissolving solution reservoir 18 while the microneedles 51 simultaneously pierce the horny layer of the skin by the pressing. The drug, now dissolved in the dissolving solution 16, is absorbed transdermally. In this example, the drug is not in the coating carrier, but is contained in the pad portion 41 (drug retainer). However, it can instead be contained in the coating carrier.

Figure 4 is a diagram of another example of a transdermal drug administration apparatus with microneedles of the present invention. The symbols in Figure 4 that are common to Figures 2 and 3 have the same meaning as in Figures 2 and 3. This example is different from the example shown in Figure 3 in that the pad portion 41 containing the drug in Figure 3 is separated into two parts, an absorbent 31 that does not contain the drug and a drug retaining material (drug retainer) 32, which contains the drug, and in that electrode 25 is provided above the absorbent (pad portion) 31 for supplying electrical energy from outside the apparatus. The lead 26 is connected to the electrode 25. By this arrangement, the apparatus of this example can be used as an electrical drug administration system like an apparatus for an iontophoresis system (an iontophoresis electrode structure) described, for instance, in Japanese Patent Laid-open No. 2003-93521. The remaining parts are the same as in Figures 2 and 3. Here, instead of the electrode 25 arranged for supplying electrical energy from the outside, a sonic transducer (not shown in the Figure) can be arranged for supplying sonic vibration energy from the outside, to use the apparatus as a sonophoresis device.

### Examples

Examples of the present invention are described below in detail. However, the present invention is not limited by the below-given examples. In all these experiments, the microneedles used were made of silicon and had a height of about 250 µm (230 to 270 µm), and a microneedle substrate (1 cm²) with 400 or 841 microneedles/cm² as a value of standard was used. A piece of foam tape (#9773, 7.84 cm²) of 3M Company was pasted on the back side of the microneedle substrate in such a way that the adhesive layer of the tape would face the skin. The projecting ends of the tape were attached to the skin to bring the microneedle side of the microneedle substrate in close contact with the skin. To start the experiment, the microneedle substrate was placed on the skin and pressure applied (2 kg/patch for 5 seconds) on the substrate with a finger.

### (Example 1)

### (Screening of water-soluble polymers for coating carriers)

Aqueous solutions each containing 5 wt.% of a polymer (polyvinyl alcohol 220, dextrin, chondroitin A, polyethylene glycol, polyvinylpyrrolidone, hydroxypropyl methylcellulose or methylcellulose), and 7 wt.% sodium calcein were prepared as coating carriers. Microneedles (400 pile/patch) were coated all over their surface with 25 µl/patch of one of these coating carriers, and dried for 30 minutes in a drier for fixing.

Skin was then removed from the trunk of a hairless mouse and fitted to a vertical acrylic cell (2.54 cm²) with the dermis side facing the receptor layer, and the whole assembly was placed in a constant temperature chamber set at 37°C. Then, the transdermal drug administration apparatus with microneedles of the present invention was pasted on the horny layer side, and hourly sampling was done for 6 h. Phosphate buffer solution (PBS) was used for the receptor layer. The drug content of the receptor solution at each time of sampling was measured by fluorescence spectrophotometry (Excitation: 485 nm, fluorescence: 538 nm).
- Animal species: Hairless mouse (n=3)
- Receptor solution: 4 mL PBS (Sampling volume: 200 µl)
- Temperature : 37°C
- Area: 2.54 cm² (The MN substrate itself was 1 cm²)

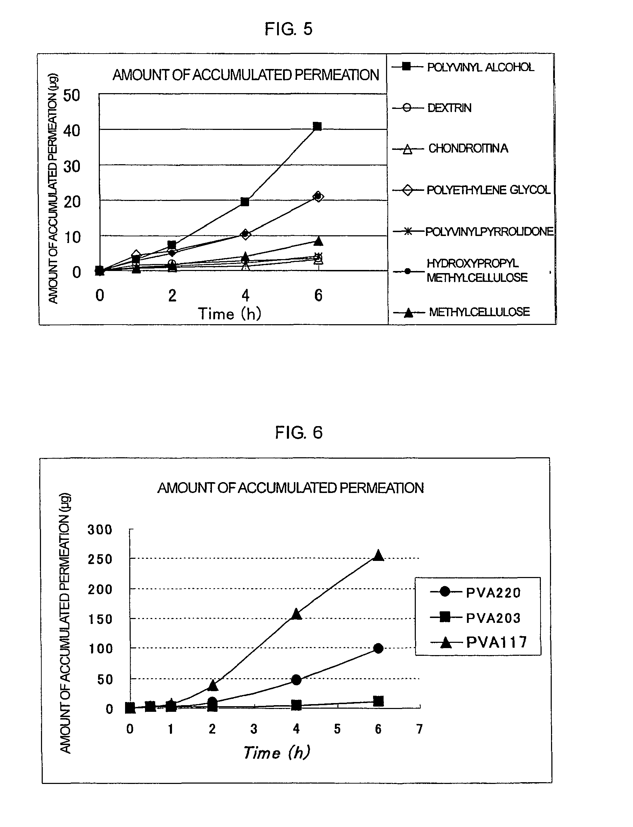

Figure 5 is a graph showing the results of measurements made in Example 1. The abscissa is time (h) and the ordinate is the cumulative drug permeation (µg). As shown in the Figure, the permeability of calcein through the skin generally increased by the addition of a polymer to the solution. Among these polymers, polyvinyl alcohol 220 caused the highest increase in permeation.

### (Example 2)

### (Screening of polyvinyl alcohol coating carrier-saponification degree)

Aqueous solutions containing 5% by weight of a polyvinyl alcohol (PVA220, PVA203, or PVA117), and 7% by weight of sodium calcein were prepared as coating carriers. Microneedles (800 pile/patch) were coated all over the surface with 30 µl/patch of one of these coating carriers, and dried for 30 minutes in a drier for fixing. Skin permeation test was carried out as in Example 1, with hairless rats (n=3).
- PVA220: saponification degree (87 to 89 mol%)
- PVA203: saponification degree (87 to 89 mol%)
- PVA117: saponification degree (97 mol% or more)

Figure 6 is a graph showing the results of measurements made in Example 2. The abscissa is time (h) and the ordinate is the cumulative drug permeation (µg). As shown in the Figure, among the different polyvinyl alcohols, PVA117 (a fully saponified substance) caused the highest increase in permeability through the skin.

### (Example 3)

### (Screening of polyvinyl alcohol coating carrier-Mean degree of polymerization)

Aqueous solutions containing 5% by weight of a polyvinyl alcohol (PVA105, PVA117, or PVA124), and 7% by weight of sodium calcein were prepared as coating carriers. Microneedles (800 pile/patch) were coated all over the surface with 30 µl/patch of one of these coating carriers, and dried for 30 minutes in a drier for fixing. Skin permeation test was carried out with hairless rats (n=3) as in Example 1.
P VA105: Mean degree of polymerization (N=500)
P VA117: Mean degree of polymerization (N=1700)
P VA124: Mean degree of polymerization (N=2400)

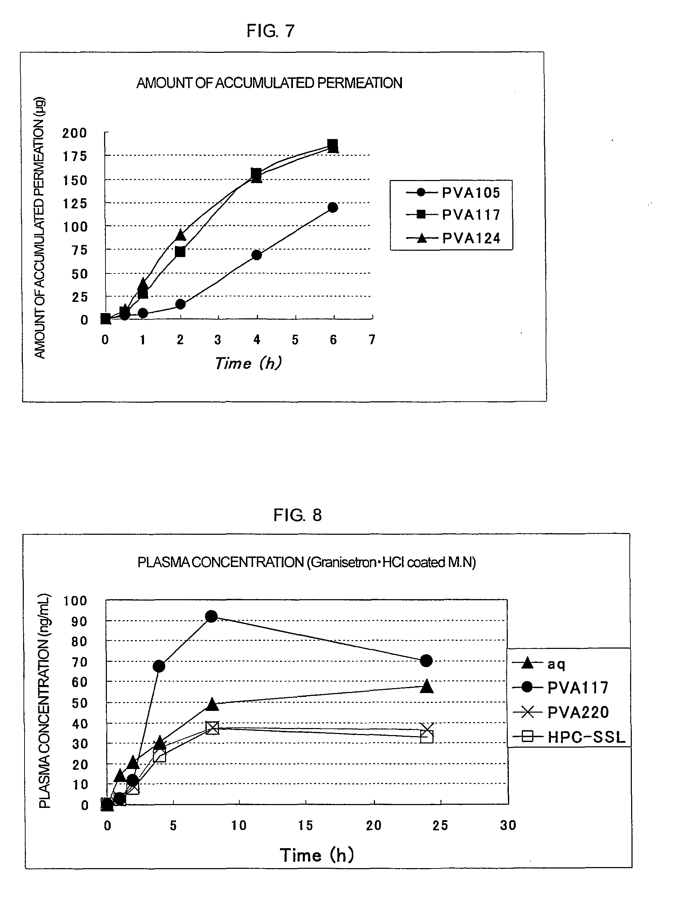

Figure 7 is a graph showing the results of measurements made in Example 3. The abscissa is time (h) and the ordinate is the cumulative drug permeation (µg). As shown in the Figure, among the polyvinyl alcohols with different degrees of polymerization, the ones with mean degree of polymerization 1700 (PVA117) and 2400 (PVA124) caused increase in skin permeability compared the one with degree of polymerization 500 (PVA117).

### (Example 4)

### (In vivo absorption (plasma concentration) test using granisetron)

Coating carriers were prepared by dissolving 16 wt.% granisetron hydrochloride in a 5 wt.% aqueous polymer solution. Microneedles (800 pile/patch) were coated all over the surface with 30 µl/patch of the coating carrier, and dried for 12 h at room temperature for fixing. In vivo testing was done with hairless rats, and blood sampled periodically was analyzed quantitatively by HPLC.
- Animal species: Hairless rat (n=4)
- Volume of blood sampled: 500 µl (plasma: 200 µl)
- HPLC measurement (Excitation: 298 nm, fluorescence: 353 nm)
- Column: TSKgel ODS-80TsQA 5 µm (4.6x150)

Figure 8 is a graph showing the results of measurements made in Example 4. The abscissa is time (h) and the ordinate is the plasma concentration (ng/ml). In this example, the low molecular weight compound used was granisetron hydrochloride, and the effect of polyvinyl alcohol was verified in vivo. As shown in the Figure, the skin permeability was higher with PVA117 grade than when no polymer was used (aq), or a soluble hydroxypropyl cellulose (HPC-SSL) or PVA220 was used.

### (Example 5)

### (Evaluation of the performance of blister-type drug product, using granisetron)

In this experiment, the coating carrier was prepared for the entire surface of microneedles by using only 5 wt.% polyvinyl alcohol (PVA117), and the microneedles (800 pile/patch) were coated all over the surface with 30 µl/patch and dried for 12 h at room temperature for fixing. After piercing the skin with the microneedles, 15 µl of 32 wt.% aqueous solution of granisetron hydrochloride, 30 µl, was applied through the through-holes (openings) on the microneedle substrate. There were two control groups. In one of these, the microneedles were not given any coating and 30 µl of the drug solution alone was applied through the through-holes. In the other control group, an aqueous solution containing 5 wt.% of polyvinyl alcohol and 32 wt.% of granisetron hydrochloride was prepared, as before, as the coating carrier, and the microneedles (800 piles/patch) were coated all over the surface with 15 µl/patch of this coating carrier.

Skin was then removed from the trunk of a hairless rat and fitted to a vertical acrylic cell (2.54 cm²) with the dermis side facing the receptor layer, and the whole assembly was placed in a constant temperature chamber set at 37°C. The transdermal drug administration apparatus with microneedles of the present invention was pasted on the horny layer side, hourly sampling was done up to 24 h. Phosphate buffer solution (PBS) was used for the receptor layer. The drug content of the receptor solution obtained at each time of sampling was measured by HPLC (Excitation: 298 nm, fluorescence: 353 nm).
- Animal species: Hairless rat (n=3)
- Receptor solution: 4 mL PBS (Sampling volume: 200 µl)
- Temperature: 37°C
- Area: 2.54 cm² (The MN substrate itself was 1 cm²)
- Column: TSKgel ODS-80TsQA 5 µm (4.6x150)

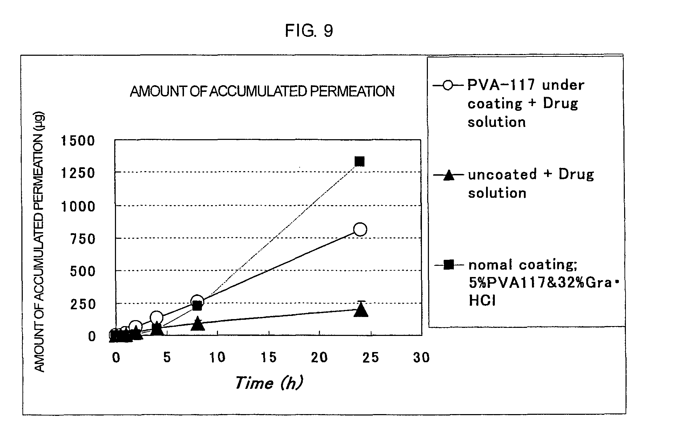

Figure 9 is a graph showing the results of measurements made in Example 5. The abscissa is time (h) and the ordinate is the cumulative drug permeation (µg). As shown in the Figure, the amount of the permeation was greater not only when a mixture of PVA117 and the drug was used for the coating (normal coating) but also when polyvinyl alcohol alone was used for the coating, and the drug was administered separately (PVA117 under coating + drug solution), compared to the case with no coating (uncoated + drug solution). The results suggest the usefulness of the coating containing PVA117.

### (Example 6)

### (Solubility of polyvinyl alcohol with 94.5 mol% or more saponification degree)

A 5 wt.% solution of a polymer (PVP, polyethyleneoxide, hydroxypropyl cellulose, PVA220, hydroxypropyl methyl cellulose, or PVA117) and 7 wt.% solution of sodium calcein, used as a model low molecular weight compound, were prepared and mixed. Fifteen ml of the mixed solution was filled in a Petri dish by the casting method and dried for 1 day at 50°C to allow a thin film to form. A 2 cm² piece of this thin film was then cut out and immersed in phosphate buffer solution (PBS) and the model compound released into the PBS solution was measured periodically. This experiment was carried out at 37°C. Table 1 shows the time of dissolution of the polymer and the time taken to reach steady state in Comparative Examples 1 to 5, and in Example 6-1 (PVA117, a fully saponified PVA of saponification degree 97 mol% or more) and Example 6-2 (PVA617, a partially saponified PVA of saponification degree 94.5 to 95.5 mol%).

**Table 1**

| | Water-soluble polymer | Dissolution time | Time to reach steady state |
|---|---|---|---|
| Comparative Example 1 | PVP | About 5 minutes | About 2 minutes (about 100%) |
| Comparative Example 2 | Polyethylene oxide | About 5 minutes | About 5 minutes (about 100%) |
| Comparative Example 3 | Hydroxypropyl cellulose | About 5 to 10 minutes | About 10 minutes (about 100%) |
| Comparative Example 4 | PVA220 | 5 to 10 minutes | About 10 minutes (about 100%) |
| Comparative Example 5 | Hydroxypropyl methylcellulose | 5 to 10 minutes | About 10 minutes (about 100%) |
| Example 6-1 | PVA117 | Not dissolve | About 10 minutes (about 100%) |
| Example 6-2 | PVA617 | Not dissolve (swollen) | About 10 minutes (about 100%) |

As shown in Table 1, all the polymers other than PVA117 (Example 6-1) and PVA617 (Example 6-2) dissolved within 10 minutes from the start of soaking, but both PVA617 and PVA117 retained the film shape even after 120 minutes and up to 12 h, although PVA617 showed some swelling. It thus became clear that PVA117 and PVA617 can not only function as drug carriers but also as routes of drug permeation via microneedles.

### (Example 7)

### (Skin permeation tests with a drug, in its free and salt forms, using hairless rats)

10 wt.% aqueous solutions of polyvinyl alcohol (PVA117) containing 16 wt.% of a drug (pergolide, pramipexol, or bisoprolol) in its free form or in the form of salt (pergolide mesylate, pramipexol hydrochloride, or bisoprolol fumarate) were prepared as the coating carrier. Microneedles (800 piles/patch) were coated all over their surfaces with 30 µl/patch one of the coating carriers, and dried at room temperature for 12 h for fixing. Skin removed from hairless rats was pierced with microneedles coated with the drug formulations, including those having their free forms, and samples were removed periodically. Phosphate buffer solution (PBS) was used for the receptor layer. The receptor solution sample, sampled at different time points, and acetonitrile were mixed at 1:1 ratio, stirred, centrifuged (15,000 rpm, 5°C, 5 minutes), then the supernatant was recovered, and its drug content measured by HPLC. Table 2 lists the maximum flux of each drug in the free form and the salt form.
- Animal species: Hairless rat (n=3)
- Sample volume: 1ml
- HPLC measurement

<Pergolide> TSKgel ODS-80TsQA(4.6x150 mm), 223 nm, 40°C
<Pramipexol> TSKgel ODS-80TsQA(4.6x150 mm), 265 nm, 40°C
<Bisoprolol> TSKgel ODS-80TsQA(4.6x150 mm), 280 nm, 40°C

**Table 2**

| Drug | Salt (µg/cm²/hr) | Free form (µg/cm²/hr) |
|---|---|---|
| Pergolide | 0.1< | 0.1< |
| Pramipexol | 180 | 100 |
| Bisoprolol | 90 | 60 |

In the case of pergolide, the amount of drug permeation was about 1 µg, a generally low value, for both the salt and the free form, in the skin permeation test. This is because this drug, whether in the free or the salt form, has almost no solubility in water. Therefore, it is assumed that the drug in the polymer did not get dissolved and did not permeate through the skin. Pramipexol and bisoprolol showed higher maximum flux in their salt form than in their free form, in the skin permeation test. Regarding this aspect, it is generally known that in the case of drug products in the form of tape formulations, etc, which do not affect the horny layer, the physicochemical properties of the drug have a major effect on skin permeability. Especially, drugs with a relatively high fat solubility have a higher permeability than highly water-soluble drugs. However, when the device of the present invention was used, the salt-form compound, which is more water-soluble than the highly fat-soluble free form, showed higher skin permeability. These results confirmed that high skin permeability can be expected even with highly water-soluble drugs when used with the device of the present invention, as can be understood from the fact that granisetron hydrochloride showed good skin permeability in examples 4 and 5.

In the experiment (Table 2) with bisoprolol, a low melting point drug that is liquid at room temperature, both the fumarate and the free form showed good skin permeability. It became clear from this result that the state, i.e., whether dissolved or crystalline, rather than the physicochemical properties of the drug, has a major impact in skin permeation performance of drug administered with the device. In other words, it is believed that skin permeability is promoted if the drug maintains its dissolved state, or gets shifted to the dissolved state, at the time of administering the drug formulation. In other words, it became clear that the water-soluble drugs so far considered not applicable in ordinary transdermal formulations have now become applicable, unless the drug has extremely low solubility, like pergolide.

### Industrial Applicability

The present invention relates to a microneedle device having, on a substrate, a plurality of microneedles that can pierce the skin for administering a drug through the skin, and a transdermal drug administration apparatus with microneedles. The invention has industrial applicability.

## Claims

1. A microneedle device comprising a plurality of microneedles on a substrate, which are capable of piercing a skin, wherein the surface of the microneedles and/or the substrate is partly or entirely coated in fixed state with a coating carrier containing polyvinyl alcohol,
wherein the coating carrier contains a drug having a molecular weight of 1000 or less, the coating carrier acting as a route for drug permeation so that the drug is transdermally delivered, and
wherein the polyvinyl alcohol has a saponification degree of 94.5 mol% or more.

2. The microneedle device according to claim 1, wherein the coating carrier maintains fixed state, without completely dissolving even after the transdermal application.

3. A transdermal drug administration apparatus with microneedles, having a microneedle device according to claim 1.

4. The transdermal drug administration apparatus with microneedles according to claim 3, further comprising a dissolving solution reservoir containing a drug solution or a dissolving solution for drug dissolution above the microneedle device.

5. A transdermal drug administration apparatus with microneedles, having a microneedle device according to claim 1 and a drug retainer retaining a drug, which is arranged above the microneedle device.

6. The transdermal drug administration apparatus with microneedles according to claim 5, further comprising a dissolving solution reservoir containing a drug solution or a dissolving solution for drug dissolution above the drug retainer.

7. The transdermal drug administration apparatus with microneedles according to any one of claims 3 to 6, further comprising an electrode for supplying electrical energy from the outside.

8. The transdermal drug administration apparatus with microneedles according to any one of claims 3 to 6, further comprising a sonic transducer for supplying sonic vibration energy from the outside.

9. A method of coating a microneedle device comprising a plurality of microneedles on a substrate, which are capable of piercing a skin, comprising the steps of coating the surface of the microneedles and/or the substrate partly or entirely with a coating carrier containing polyvinyl alcohol, and drying and fixing the coating carrier thereto
wherein the coating carrier contains a drug having a molecular weight of 1000 or less, the coating carrier acting as a route for drug permeation so that the drug is transdermally delivered, and
wherein the polyvinyl alcohol has asaponification degree of 94.5 mol% or more.

10. The method of coating the microneedle device according to claim 9, wherein, before fixing the coating carrier, the polyvinyl alcohol has a mean degree of polymerization of 200 to 3,500.

## Patentansprüche

1. Mikronadelvorrichtung, umfassend eine Vielzahl von Mikronadeln auf einem Substrat, welche zum Durchstechen einer Haut in der Lage sind, wobei die Oberfläche der Mikronadeln und/oder des Substrats teilweise oder vollständig in fixiertem Zustand mit einem Beschichtungsträger, welcher Polyvinylalkohol enthält, beschichtet ist,
wobei der Beschichtungsträger einen Arzneistoff mit einem Molekulargewicht von 1000 oder niedriger enthält, wobei der Beschichtungsträger als ein Weg zur Arzneistoffpermeation fungiert, so dass der Arzneistoff transdermal bereitgestellt wird, und
wobei der Polyvinylalkohol einen Verseifungsgrad von 94,5 Mol-% oder höher aufweist.

2. Mikronadelvorrichtung gemäß Anspruch 1, wobei der Beschichtungsträger den fixierten Zustand beibehält, ohne vollständiges Lösen sogar nach der transdermalen Applikation.

3. Gerät zur transdermalen Arzneistoffverabreichung mit Mikronadeln, welches eine Mikronadelvorrichtung gemäß Anspruch 1 aufweist.

4. Gerät zur transdermalen Arzneistoffverabreichung mit Mikronadeln gemäß Anspruch 3, ferner umfassend ein Reservoir für eine lösende Lösung, welches eine Arzneistofflösung oder eine lösende Lösung zum Arzneistofflösen enthält, über der Mikronadelvorrichtung.

5. Gerät zur transdermalen Arzneistoffverabreichung mit Mikronadeln, welches eine Mikronadelvorrichtung gemäß Anspruch 1 und eine Arzneistoffrückhaltevorrichtung, die einen Arzneistoff zurückhält und über der Mikronadelvorrichtung angeordnet ist, aufweist.

6. Gerät zur transdermalen Arzneistoffverabreichung mit Mikronadeln gemäß Anspruch 5, ferner umfassend ein Reservoir für eine lösende Lösung, welches eine Arzneistofflösung oder eine lösende Lösung zum Arzneistofflösen enthält, über der Arzneistoffrückhaltevorrichtung.

7. Gerät zur transdermalen Arzneistoffverabreichung mit Mikronadeln gemäß einem der Ansprüche 3 bis 6, ferner umfassend eine Elektrode zum Zuführen von elektrischer Energie von außen.

8. Gerät zur transdermalen Arzneistoffverabreichung mit Mikronadeln gemäß einem der Ansprüche 3 bis 6, ferner umfassend einen Schallwandler zum Zuführen von Schallschwingungsenergie von außen.

9. Verfahren zum Beschichten einer Mikronadelvorrichtung, umfassend eine Vielzahl von Mikronadeln auf einem Substrat, welche zum Durchstechen einer Haut in der Lage sind, umfassend die Schritte von Beschichten der Oberfläche der Mikronadeln und/oder des Substrats teilweise oder vollständig mit einem Beschichtungsträger, welcher Polyvinylalkohol enthält, und Trocknen und Fixieren des Beschichtungsträgers daran,
wobei der Beschichtungsträger einen Arzneistoff mit einem Molekulargewicht von 1000 oder niedriger enthält, wobei der Beschichtungsträger als ein Weg zur Arzneistoffpermeation fungiert, so dass der Arzneistoff transdermal bereitgestellt wird, und
wobei der Polyvinylalkohol einen Verseifungsgrad von 94,5 Mol-% oder höher aufweist.

10. Verfahren zum Beschichten einer Mikronadelvorrichtung gemäß Anspruch 9, wobei vor dem Fixieren des Beschichtungsträgers der Polyvinylalkohol einen mittleren Polymerisationsgrad von 200 bis 3.500 aufweist.

## Revendications

1. Dispositif à micro-aiguilles comprenant une série de micro-aiguilles sur un substrat, lesquelles sont capables de percer la peau, où la surface des micro-aiguilles et/ou du substrat est partiellement ou entièrement enduite d'un revêtement-support stable contenant un poly(alcool vinylique),
où le revêtement-support contient un médicament ayant un poids moléculaire de 1000 ou moins, le revêtement-support agissant comme voie pour la pénétration du médicament de sorte que le médicament est administré de manière transdermique, et
où le poly(alcool vinylique) présente un degré de saponification de 94,5% en moles ou plus.

2. Dispositif à micro-aiguilles selon la revendication 1, où le revêtement-support conserve son état de manière stable, sans se dissoudre complètement, même après l'administration transdermique.

3. Appareil d'administration transdermique d'un médicament avec micro-aiguilles, ayant un dispositif à micro-aiguilles selon la revendication 1.

4. Appareil d'administration transdermique d'un médicament avec micro-aiguilles selon la revendication 3, comprenant en outre, un réservoir de solution de dissolution contenant une solution du médicament ou une solution de dissolution pour le médicament, au-dessus du dispositif à micro-aiguilles.

5. Appareil d'administration transdermique d'un médicament avec micro-aiguilles, ayant un dispositif à micro-aiguilles selon la revendication 1 et un récipient de retenue de médicament, retenant un médicament, qui se trouve au-dessus du dispositif à micro-aiguilles.

6. Appareil d'administration transdermique d'un médicament avec micro-aiguilles selon la revendication 5, comprenant en outre, un réservoir de solution de dissolution contenant une solution du médicament ou une solution de dissolution pour le médicament, au-dessus du dispositif à micro-aiguilles.

7. Appareil d'administration transdermique d'un médicament avec micro-aiguilles selon l'une quelconque des revendications 3 à 6, comprenant en outre, une électrode pour l'alimentation en énergie électrique depuis l'extérieur.

8. Appareil d'administration transdermique d'un médicament avec micro-aiguilles selon l'une quelconque des revendications 3 à 6, comprenant en outre, un transducteur sonore pour l'alimentation en énergie de vibration sonore depuis l'extérieur.

9. Procédé d'enduction d'un dispositif à micro-aiguilles, comprenant une série de micro-aiguilles sur un substrat, lesquelles sont capables de percer la peau, comprenant l'étape d'enduction de la surface des micro-aiguilles et/ou du substrat, partiellement ou entièrement avec un revêtement-support stable contenant un poly(alcool vinylique), le séchage et la fixation du revêtement -support,
où le revêtement-support contient un médicament ayant un poids moléculaire de 1000 ou moins, le revêtement-support agissant comme voie pour la pénétration du médicament de sorte que le médicament est administré de manière transdermique, et
où le poly(alcool vinylique) présente un degré de saponification de 94,5% en moles ou plus.

10. Procédé d'enduction du dispositif à micro-aiguilles selon la revendication 9, où, avant de fixer le revêtement -support, le poly(alcool vinylique) a un degré moyen de polymérisation allant de 200 à 3500.
